# EUROPEAN PATENT SPECIFICATION

(11) **EP 3 283 093 B1**
(45) Date of publication and mention of the grant of the patent: **16.09.2020**
(21) Application number: 15717170.3
(22) Date of filing: 17.04.2015
(51) Int. Cl.: A61K 36/81, A61K 36/886, A61K 38/56, A61K 9/00, A61K 45/06, A61K 31/14, A61K 31/164, A61K 31/415, A61K 31/695, A61K 31/7048, A61K 33/30, A61K 36/28, A61K 36/48, A61K 9/06, A61K 31/4174, A61P 17/00

(54) **COMBINATION OF AT LEAST ONE PROTEASE INHIBITOR AND DEXPANTHENOL FOR USE IN THE PREVENTION AND/OR TREATMENT OF SKIN LESIONS**
KOMBINATION AUS MINDESTENS EINEM PROTEASEINHIBITOR UND DEXPANTHENOL ZUR VERWENDUNG BEI DER VORBEUGUNG UND/ODER BEHANDLUNG VON HAUTVERLETZUNGEN
COMBINAISON D'AU MOINS UN INHIBITEUR DE PROTÉASE ET DU DEXPANTHÉNOL POUR UNE UTILISATION DANS LA PRÉVENTION ET/OU LE TRAITEMENT DE LÉSIONS CUTANÉES

(43) Date of publication of application: 21.02.2018
(73) Proprietor: Eurodrug Laboratories B.V., 2516 AC The Hague (NL)
(72) Inventor: ASSANDRI, Alessandro, CH-6850 Mendrisio (CH)
(74) Representative: Pistolesi, Roberto
(86) International application number: PCT/EP2015/058451
(87) International publication number: WO 2016/165783

(56) References cited:
- EP-A1- 1 586 336
- EP-A1- 1 736 136
- WO-A1-99/59623
- US-A1- 2003 077 307
- US-A1- 2005 175 719
- EBNER FRITZ ET AL: "Topical use of dexpanthenol in skin disorders", AMERICAN JOURNAL OF CLINICAL DERMATOLOGY, ADIS, US, vol. 3, no. 6, 1 January 2002 (2002-01-01) , pages 427-433, XP009181863, ISSN: 1175-0561, DOI: 10.2165/00128071-200203060-00005
- "Dexpanthenol", INTERNET CITATION, [Online] 1 January 2009 (2009-01-01), pages 1-2, XP003025923, Retrieved from the Internet: URL:http://de.wikipedia.org/wiki/Dexpanthe nol> [retrieved on 2009-11-12]

## Description

### BACKGROUND OF THE INVENTION

Diaper rash is a common form of inflamed skin (dermatitis) that appears as a patchwork of bright red skin on baby's bottom and also as peri-anal dermatitis in adults with urine and/or faeces incontinence, in non- autonomous elderly patients, in long term hospitalized patients, and in bed-confined patients with sores.

Diaper rash is commonly linked to continuously wet or infrequently changed diapers, diarrhoea, and using plastic pants to cover diapers. Diaper rash also may develop after solid foods are added to baby's diet, when breast-feeding mothers eat certain foods, when the baby is changing from mother's milk to milk formula, when changing from one type of milk formula to another, during teething, or when a baby is taking antibiotics (1).

Diaper rash is characterized by
- skin signs: diaper rash is marked by red, puffy and tender-looking skin in the diaper region - buttocks, thighs and genitals, which may even be bleeding.
- changes in baby's disposition: baby with a diaper rash often fusses or cries when the diaper area is washed or touched.

Diaper rashes in babies can occur intermittently, anytime a child wears diapers, but they're more common in babies during their first 15 months, especially between 9 and 12 months of age. Growing up, babies and toddlers up to four years, often develop rash during diaper use for the night.

The wearing of diapers causes a significant increase in skin wetness and pH. Prolonged wetness leads to softening of the stratum corneum, the outer, protective layer of the skin. Weakening of its physical integrity makes the stratum corneum more susceptible to damage by friction from the surface of the diaper, and local irritants. The main irritants in this situation are faecal proteases and lipases, whose activity is increased greatly by elevated pH (2).

Faeces contain proteases such as trypsin, α-chymotrypsin and elastases which are secreted by the pancreas, the small intestinal brush-border and the stomach. The proteolytic activity in faeces is several times higher in newborns and infants than in adults (3).

Protease inhibitors are molecules that inhibit the function of proteases. In particular, faecal protease inhibitors can be of different origins, such as plants, fruits, rice bran, cowpea seeds, soybean or potato (9, 10, 11).

Potato derived protease inhibitors have been disclosed and isolated in WO2008/069650, where they have been shown as having a broad range of potentially important applications, for instance as a gelling or foaming agent in a food or industrial product or in different therapeutic applications. Furthermore, WO99/059623 discloses the use of protease inhibitors derived from plants, such as fruits, rice bran, cowpea seeds, soybean or potato, in the treatment of diabetes, in reducing skin cancer, for inhibiting the growth of bacteria and for preventing or treating inflammation or pruritus of skin and intestine. In an *in vitro* assay (4) the inhibition of faecal proteolytic activity by potato proteins was investigated. The inhibition was established by adding increasing concentrations, 0.5-10%, of potato proteins to faeces from patients with different intestinal diseases and faeces from healthy infants. Crude and more purified protein fractions with protease inhibitor activity were prepared from potatoes. Both fractions were tested with faeces from subjects with high proteolytic activity, patients with an ileostomy, patients with an ileum reservoir, patients after colectomy with ileoanal anastomosis (IAA) and healthy infants of 4 months old. It has been observed that both fractions were able to inhibit the majority of protease activity in each of the faecal samples , however, the second fraction was more effective.

The activity of potato proteins in preventing protease-induced skin irritation (dermatitis) has also been demonstrated *in vivo* on the skin of human volunteers (4). In a different study in children after resection of long-segment Hirschsprung's disease (a condition that affects the large intestine and causes problems with passing stool), with severe perianal dermatitis (SPAD) due to high stool frequency and elevated concentrations of faecal pancreatic proteases, the effect of potato-derived protease inhibitors on skin conditions was investigated. No adverse effects were observed after treatment with the protease inhibitors. The results of the study suggest that protease inhibitors may reduce otherwise intractable protease-induced skin irritation in infants (3).

Potatoes, rice bran, cowpea seeds and soybean are natural products that are widely used in the daily diet, therefore allergenic reactions to the related derived protease inhibitors are highly unlikely.

For example, the possibility of adverse reactions to potato proteins was investigated by challenging subjects with a history of type I and type IV hypersensitivity. None of the 63 patients suffering from allergic contact dermatitis (type IV), who were challenged with purified potato proteins, showed a skin reaction. Thirteen patients suffering from food allergic (type I) reactions, including the patient with complaints during contact with raw potatoes and a positive skin reaction after challenge with extract from raw potatoes, did not show any reaction to the skin (4).

Panthenol and dexpanthenol-containing creams have been widely used for treatment of lesions (superficial wounds) of the skin and mucous membranes. Dexpanthenol is converted in tissues to pantothenic acid, a component of coenzyme A. Coenzyme A catalyses early steps in the synthesis of fatty acids and sphingolipids which are of crucial importance for stratum corneum lipid bilayers and cell membrane integrity. This enables dexpanthenol to significantly accelerate skin barrier repair and stratum corneum hydration, while reducing skin roughness and inflammation (5).

Zinc oxide is a mildly antiseptic substance that is used topically in different therapeutic applications. It is active enough to protect skin from infections without inducing an adverse reaction, for this reasons it is used in powders, creams and ointments, particularly for babies. This application of zinc oxide is one of the main use of zinc and its value in this respect has been known for millennia.

Benzalkonium chloride is used as an active ingredient in many consumer products. In pharmaceutical products it can be used as a preservative, sanitizer, antiseptic and disinfectants in personal care, skin, hair and cosmetic products. The safety factor of benzalkonium chloride allows its use in a wide range of leave-on skin sanitizers and sanitary baby wipes. Benzalkonium has been used in various formulations for diaper rash.

Cetrimide is a quaternary ammonium disinfectant with properties typical of cationic surfactants. This is useful in the treatment and prevention of nappy rash, acting to suppress the development of ammonia producing organisms usually associated with this condition (7).

Clotrimazole is indicated for the treatment of skin infections due to dermatophytes (e.g. trycophyton species), yeasts (e.g. candida species), moulds and other fungi. These include ringworm (tinea) infections, paronychia, pityriasis versicolor, erythrasma and intertrigo, as well as fungal nappy rash, candidal vulvitis and candidal balanitis. Clotrimazole has a broad antimycotic spectrum of action *in vitro* and *in vivo,* which includes dermatophytes, yeasts, moulds, etc. The mode of action of clotrimazole is fungistatic or fungicidal depending on the concentration of clotrimazole at the site of infection.

Nystatin is a polyene antifungal antibiotic that interferes with the permeability of the cell membrane of sensitive fungi by binding to sterols, chiefly ergosterol. Nystatin is used for the prophylaxis and treatment of candidiasis of the skin. It is both fungistatic and fungicidal against a wide range of yeasts and yeast-like fungi (8).

Dimeticones and other silicones are water-repellent and have a low surface tension. They are used in topical barrier preparations, for instance for diaper rash, for protecting the skin against water-soluble irritants (8).

Extracts from *Aloe vera* are widely used in the cosmetics industry, being marketed as having rejuvenating, healing, or soothing properties. *Aloe vera* leaves contain a clear gel that is often used as a topical ointment, e.g. for osteoarthritis, burns, sunburns, and psoriasis. *Aloe vera* gel can be found in hundreds of skin products, including lotions and sunblocks. The many uses of *Aloe vera* make it an excellent ingredient as a multipurpose skin treatment and is useful as a co-adjuvant for its healing and soothing properties in topical formulations for diaper rash.

*Calendula* species have been used traditionally as culinary and medicinal herbs. The flowers were applied to cuts and wounds to stop bleeding, prevent infection and speed healing. *Calendula* has been shown to help wounds heal faster, possibly by increasing blood flow and oxygen to the affected area, which helps the body grow new tissue. It is also used to improve skin hydration and firmness. The oil of *Calendula officinalis* is used as an anti-inflammatory and a remedy for healing wounds and also used topically for treating acne, reducing inflammation, controlling bleeding, and soothing irritated tissue. Limited evidence indicates calendula cream or ointment is effective in treating radiation dermatitis. For its anti-inflammatory effect, soothing and wound healing properties *calendula* is a useful ingredient in topical formulations for diaper rash.

Nowadays the treatment of faeces-induced inflammations is mainly based on providing either a protective layer to the skin, e.g. by applying a lipid-based ointment, containing additives such as zinc or aluminium, making a barrier, or by applying a general anti-inflammatory agent, i.e. topical corticosteroids.

Furthermore, potato protease inhibitors have shown an effective activity in neutralizing the activity of pancreatic protease in order to prevent skin injury in diaper rash.

However, when used alone all of the above mentioned ingredients only provide relief of the manifestation of diaper rash clinical symptoms.

It is therefore evident that there is a need to find one or more compositions that are effective in the prevention and/or treatment of skin damages due to diaper rash and as adjuvant in skin repair, in children and other adult patients.

### DEFINITION

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term "physiologically/pharmaceutically acceptable excipient" herein refers to a substance devoid of any pharmacological effect of its own and which does not produce adverse reactions when administered to a mammal, preferably a human. Physiologically/ pharmaceutically acceptable excipients are well known in the art and are disclosed, for instance in the Handbook of Pharmaceutical Excipients, sixth edition 2009.

The term "pharmaceutically acceptable salts or derivatives" herein refers to those salts or derivatives which possess biological effectiveness and properties of the salified compound and which do not produce adverse reactions when administered to a mammal, preferably a human. The pharmaceutically acceptable salts may be inorganic or organic salts; examples of pharmaceutically acceptable salts include but are not limited to: carbonate, chloride, hydrobromide, sulphate, hydrogen sulphate, citrate, maleate, fumarate, trifluoroacetate, 2-naphthalenesulphonate, and paratoluenesulphonate. Further information on pharmaceutically acceptable salts can be found in Handbook of pharmaceutical salts, P. Stahl, C. Wermuth, WILEY-VCH, 127-133, 2008.

The pharmaceutically acceptable derivatives include the esters, the ethers and the N-oxides.

The terms "comprising", "having", "including" and "containing" are to be construed as open-ended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as semi-closed terms, meaning that no other ingredients which materially affect the basic and novel characteristics of the invention are included (optional excipients may thus be included).

The terms "consists of", "consisting of" are to be construed as a closed term.

The term "simultaneous, separate or sequential administration" herein refers to administration of the first and second compounds at the same time or in such a manner that the two compound act at the same time or administration of one compound after the other compound in such a manner to provide a therapeutic effect.

### DETAILED DESCRIPTION OF THE INVENTION

It has now surprisingly found that a combination comprising at least one protease inhibitor and at least one additional active principle with established use in diaper rash, is particularly effective in the prevention and/or treatment of skin damages caused by proteases in the urine and/or faeces and as adjuvant in skin repair.

In particular, it has been demonstrated that the combination of a potato derived protease inhibitor and one or more active principles show an unexpected and positive advantage, with respect to the use of the single components, in treating and/or preventing diaper rash in babies and other patients.

The effect of said combination is highly superior to the effect observed for the single component when used for the same kind of treatments, showing a surprising synergistic action of the combination of the present invention.

Said effect can be observed also when the combination is associated with a further active principle. For example, it has been observed that, a combination containing a protease inhibitor, benzalkonium chloride and cetrimide, combine the protective effect of the protease inhibitors on the skin with the ability of benzalkonium chloride and cetrimide to suppress the development of ammonia producing organisms usually associated with diaper rash.

Therefore, the components of the combination together have the unique possibility to:
- inhibit the cause of the diaper rash (i.e. the proteases), thereby preventing further harm to the baby's or patient's skin.
- accelerate skin barrier repair and moisturize the skin to soothe and heal the skin.

The present invention is therefore a combination comprising at least one potato derived protease inhibitor and at least one active principle selected from dexpanthenol, its pharmaceutically acceptable salts or a mixture thereof.

In one embodiment of the present invention, the quantity of said at least one protease inhibitor or a mixture thereof is between 0.1% and 20% of the combination, preferably between 2 to 15%, more preferably between 4 to 10%, where said percentage is referred to the total weight of the combination.

According to a further preferred embodiment of the present invention, the quantity of said at least one additional active principle, their pharmaceutically acceptable salts or a mixture thereof is between 0.01% and 40% of the combination, preferably between 2 to 25%, more preferably between 4 to 15%, where said percentage is referred to the total weight of the combination.

Particularly preferred are the combinations in which the percentage amount of said protease inhibitor and of said at least one additional active principle in the combination are respectively: 5% and 13%, or 5% and 5 %, or 5% and 0.01% , or 5% and 0.5%, wherein said percentages are referred to the total weight of the combination.

A further embodiment of the present invention is a combination comprising at least one potato derived protease inhibitor and at least one additional active principle, selected from dexpanthenol, its pharmaceutically acceptable salts or a mixture thereof, for use in the prevention and/or treatment of skin damages caused by urine and/or faeces and as adjuvant in skin repair.

Preferably, said skin damages are perineal, peri-anal inflammation or dermatitis present pre-mature babies, babies and other adult patients.

In a further embodiment the combination of the present invention comprises a further additional active principle, selected from antifungals, antiseptics and disinfectants, surfactants and defoamers, skin soothing agents, anti-inflammatory, anti-bacterial, antihistamine and antioxidants or vitamins.

Preferably said antifungals are selected from nystatin, clotrimazol, hydrocortisone, miconazole, bromochloro-salicylanilide, methylrosaniline, tribromometacresol, undecylenic acid, polynoxylin, 2-(4-chlorphenoxy)-ethanol, chlorphenesin, ticlatone, sulbentine, ethyl hydroxybenzoate, haloprogin, salicylic acid, selenium sulfide, ciclopirox, terbinafine, amorolfine, dimazole, tolnaftate, flucytosine, naftifine, butenafine, more preferably nystatin or clotrimazol.

Preferably said antiseptics and disinfectants are selected from cetrimide, cromoglicic acid, ethacridine lactate, aminoacridine, euflavine, dibrompropamidine, chlorhexidine, propamidine, hexamidine, polihexanide, hexachlorophene, policresulen, phenol, triclosan, chloroxylenol, biphenylol, nitrofural, dequalinium, chlorquinaldol, oxyquinoline, clioquinol, benzalkonium, cetrimonium, cetylpyridinium, benzoxonium chloride, dodecyldimethylammonium chloride, benzethonium chloride, decamethoxine, silver, silver nitrate, more preferably cetrimide or benzalkonium.

Preferably said surfactants and defoamers are selected from dimethicone or simethicone, more preferably dimethicone.

Preferably said skin soothing agents are selected from *aloe vera, calendula,* vitamin E, retinol or ergocalciferol, more preferably *aloe vera.*

Preferably said anti-inflammatory active principles are selected from *calendula* or echinacea, more preferably *calendula.*

Preferably said anti-bacterial active principles are selected from isopropylmethylphenol or neomycin, more preferably isopropylmethylphenol.

Preferably said antihistamine active principles are selected from mepyramine maleate, diphenhydramine, thonzylamine, thenalidine, tripelennamine, chloropyramine, promethazine, tolpropamine, dimetindene, clemastine, bamipine, isothipendyl, diphenhydramine, diphenhydramine methylbromide or chlorphenoxamine, more preferably mepyramine maleate or diphenhydramine.

Preferably, said antioxidants and/or vitamins is tocopherol acetate.

A further embodiment of the present invention is a pharmaceutical composition comprising the combinations described above together with pharmaceutically acceptable excipients and/or adjuvants.

In a preferred embodiment the pharmaceutical composition of the present invention contains from 0.1% to 50% of said combination, preferably from 0.005% to 40%, more preferably from 0.01% to 20%, where said percentages are referred to the total weight of the composition.

In a further object of the present invention said pharmaceutical composition is suitable for use in the prevention and/or treatment of skin damages caused by urine and/or faeces and as adjuvant in skin repair.

Preferably, said pharmaceutical composition is for simultaneous, separate or sequential use in the prevention and/or treatment of skin damages caused by urine and/or faeces and as adjuvant in skin repair.

In a preferred embodiment said skin damages are perineal, perianal inflammation present in premature babies, babies or other adult patients.

According to a preferred embodiment of the present invention, the pharmaceutical composition comprising the combinations of the present invention together with pharmaceutically acceptable excipients and/or adjuvants is administered topically.

Preferably, the pharmaceutical form is selected from cream, ointment, gel, foam, powder, spray and gauzes, preferably cream or ointment.

According to an embodiment of the present invention, the pharmaceutical composition comprising the combinations disclosed above is administered daily, preferably one or more times per day.

According to a further preferred embodiment, said pharmaceutical composition should be applied at every diaper change, with a minimum of once a day. Preferably, the daily administration can be continued as long as diapers are used.

In a further embodiment the composition of the present invention can be administered to humans, intended as adult subject and as "paediatric population", where the term "paediatric population" identifies that part of the population from birth to eighteen years old.

For the purpose of the present invention, the compositions will be prepared according to conventional techniques, and may contain, to make up to 100%, compatible excipients and/or pharmaceutically acceptable carriers e.g. humectants (glycerin), emulsion stabilisers (xanthan gum, polyacrylate crosspolymer-11), emulsifiers (glyceryl stearate, cetearyl alcohol, sodium stearoyl glutamate), emollients (decyl oleate, petrolatum, dimethicone) or preservatives (sodium benzoate).

The pharmaceutical compositions and the uses of the present invention will now be more fully described by the following examples. It should, however, be noted that such example are given by way of illustration and not of limitation.

### Experimental data

The following compositions have been prepared.

### Example 1 (not part of the invention)

| | %W/W |
|---|---|
| Aqua | Up to volume |
| Decyl oleate | 10.00 |
| Protease inhibitor | 5.00 |
| Zinc oxide | 1.00-40.00 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Dimethicone | 0.80 |
| Sodium benzoate | 0.50 |
| Sodium stearoyl glutamate | 0.40-0.50 |
| Xanthan gum | 0.30 |

### Example 2 (not part of the invention)

| | %W/W |
|---|---|
| Aqua | Up to volume |
| Decyl oleate | 10.00 |
| Protease inhibitor | 5.00 |
| Belzalkonium chloride | 0.10 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Dimethicone | 0.80 |
| Sodium benzoate | 0.50 |
| Sodium stearoyl glutamate | 0.40-0.50 |
| Xanthan gum | 0.30 |

### Example 3 (not part of the invention)

| | %W/W |
|---|---|
| Aqua | Up to volume |
| Decyl oleate | 10.00 |
| Protease inhibitor | 5.00 |
| Cetrimide | 0.5 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Dimethicone | 0.80 |
| Sodium benzoate | 0.50 |
| Sodium stearoyl glutamate | 0.40-0.50 |
| Xanthan gum | 0.30 |

### Example 4 (not part of the invention)

| | %W/W |
|---|---|
| Aqua | Up to volume |
| Decyl oleate | 10.00 |
| Clotrimazole | 1.00 |
| Protease inhibitor | 5.00 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Dimethicone | 0.80 |
| Sodium benzoate | 0.50 |
| Sodium stearoyl glutamate | 040-0.50 |
| Xanthan gum | 0.30 |

### Example 5 (not part of the invention)

| | %W/W |
|---|---|
| Aqua | Up to volume |
| Decyl oleate | 10.00 |
| Nystatin | 3.00 |
| Protease inhibitor | 5.00 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Dimethicone | 0.80 |
| Sodium benzoate | 0.50 |
| Sodium stearoyl glutamate | 0.40-0.50 |
| Xanthan gum | 0.30 |

### Example 6 (not part of the invention)

| | %W/W |
|---|---|
| Aqua | Up to volume |
| Decyl oleate | 10.00 |
| Dimethicone | 10.0 |
| Protease inhibitor | 5.00 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Sodium benzoate | 0.50 |
| Sodium stearoyl glutamate | 0.40-0.50 |
| Xanthan gum | 0.30 |

### Example 7 (not part of the invention)

| | %W/W |
|---|---|
| Aqua | Up to volume |
| Decyl oleate | 10.00 |
| Aloe vera | 10.00 |
| Protease inhibitor | 5.00 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Dimethicone | 0.80 |
| Sodium benzoate | 0.50 |
| Sodium stearoyl glutamate | 0.40-0.50 |
| Xanthan gum | 0.30 |

### Example 8 (not part of the invention)

| | %W/W |
|---|---|
| Aqua | Up to volume |
| Decyl oleate | 10.00 |
| Calendula | 9.00 |
| Protease inhibitor | 5.00 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Dimethicone | 0.80 |
| Sodium benzoate | 0.50 |
| Sodium stearoyl glutamate | 0.40-0.50 |
| Xanthan gum | 0.30 |

### Example 9

| | %W/W |
|---|---|
| Aqua | Up to volume |
| Decyl oleate | 10.00 |
| Dexpanthenol | 5.00 |
| Protease inhibitor | 5.00 |
| Caprylic/capric triglyceride | 3.00 |
| Cetearyl alcohol | 3.00 |
| Petrolatum | 3.00 |
| Glycerin | 2.985 |
| Glyceryl stearate se | 2.00-2.80 |
| Glyceryl stearate | 1.50 |
| Polyacrylate crosspolymer-11 | 1.00 |
| Dimethicone | 0.80 |
| Sodium benzoate | 0.50 |
| Sodium stearoyl glutamate | 0.40-0.50 |
| Xanthan gum | 0.30 |

### HRIPT - Hypoallergenic study

Although all ingredients in the compositions of the Examples 1-9 (Diapo-Care®) and other formulations containing a protease inhibitor are selected for their hypoallergenic potential, a modified Draize repeat insult patch test was performed in 100 healthy volunteers, of either sex and with self-perceived sensitive skin, to investigate the irritation and sensitisation potential (6). The study was performed by Princeton Consumer Research, UK with a dosing regime of nine, 47 hour patch applications and One 47 hour challenge patch application.

The study was performed with the combination cream of potato derived protease inhibitors and dexpanthenol (as described in example 9), and also with a cream containing only potato derived protease inhibitors (as described in example 9, but without dexpanthenol) as the active ingredient to test the skin tolerability of the product and to confirm the hypoallergenic properties.

The compositions are proven to be safe and effective creams to treat and prevent diaper rash with a unique dual action which protects the skin against the cause of diaper rash and helps to heal the skin if it is already damaged.

### REFERENCES

1. Mayo Clinic. Diaper rash. http://www.mayoclinic.org/diseases-conditions/diaper-rash/basics/definition/con-20019220. Last viewed 12 December 2014.
2. Atherton DJ. A review of the pathophysiology, prevention and treatment of irritant diaper dermatitis. Curr Med Res Opin. 2004 May;20(5):645-9.
3. Berger S, Rufener J, Klimek P, Zachariou Z, Boillat C. Effects of potato-derived protease inhibitors on perianal dermatitis after colon resection for long-segment Hirschsprung's disease. World J Pediatr. 2012 May;8(2): 173-6.
4. Ruseler-van Embden JG, van Lieshout LM, Smits SA, van Kessel 1, Laman JD. Potato tuber proteins efficiently inhibit human faecal proteolytic activity: implications for treatment of peri-anal dermatitis. Eur J Clin Invest. 2004 Apr;34(4):303-11.
5. Proksch E, Nissen HP. Dexpanthenol enhances skin barrier repair and reduces inflammation after sodium lauryl sulphate-induced irritation. J Dermatolog Treat. 2002 Dec;13 (4):173-8.
6. Princeton consumer research study No.: EURRIP1
7. Drapolene Cream SPC. http://www.medicines.org.uk/emc/medicine/7158
8. Timodine Cream SPC. http://www.medicines.org.uk/emc/medicine/17934
9. Tashiro M, et al., Purification and characterization of trypsin inhibitor from rice bran. J. Nutr. Sci. Vitaminol. 1979; 25 (3): 255-264.
10. Wang J. et al. Extraction, purification and characterization of a trypsin inhibitor from cowpea seeds. Prep. Biochem. Biotechnol. 2014; 44 (1): 1-15.
11. Bijina B. et al., Protease inhibitor from Moringa olifera with potential for use as therapeutic drug and as seafood preservative. Saudi Journal of Biological Science. 2011, 18: 273-281.

## Claims

1. Combination comprising at least one potato derived protease inhibitor and dexpanthenol, its pharmaceutically acceptable salts or a mixture thereof.

2. Combination according to claim 1, wherein the quantity of said at least one potato derived protease inhibitor or a mixture thereof is between 0.1% and 20% of the combination, preferably between 5 to 15%, more preferably between 4 to 10% of the combination.

3. Combination according to claim 1, wherein the quantity of dexapanthenol, its pharmaceutically acceptable salts or a mixture thereof is between 0.01% and 40% of the combination, preferably between 2 to 25%, more preferably between 4 to 25% of the combination.

4. Combination according to claim 1 to 3 for use in the prevention and/or treatment of skin damages caused by urine and/or faeces and as adjuvant in skin repair.

5. Combination for use according to claim 4, wherein said skin damages are perineal, perianal inflammation or dermatitis present in premature babies, babies or other adult patients.

6. Combination according to claims 1 to 3 or combination for use according to claims 4 or 5, **characterized by** comprising a further additional active principle, selected from antimicotics, antiseptics and disinfectants, surfactants and defoamers, skin soothing agents, anti-inflammatory, anti-bacterial, antihistamine and antioxidants and/or vitamins.

7. Pharmaceutical composition comprising the combinations of claims 1 to 3 together with pharmaceutically acceptable excipients and/or adjuvants.

8. Pharmaceutical composition according to claim 7, **characterized in that** it contains from 0.1% to 50% of said combination preferably from 0.01% to 20%.

9. Pharmaceutical composition according to claims 7 or 8 for use in the the prevention and/or treatment of skin damages caused by urine and/or faeces and as adjuvant in skin repair.

10. Pharmaceutical composition according to claims 7 or 8, **characterized in that** it is for simultaneous, separate or sequential use in the prevention and/or treatment of skin damages caused by urine and/or faeces and as adjuvant in skin repair.

11. Pharmaceutical composition for use according to claim 9 **characterized in that** it is administered topically.

12. Pharmaceutical composition for use according to claim 11, wherein the pharmaceutical form is selected from cream, ointment, gel, foam, powder, spray and gauzes, preferably cream or ointment.

## Patentansprüche

1. Kombination, welche mindestens einen aus Kartoffeln erhaltenen Protease-Inhibitor und Dexpanthenol, deren pharmazeutisch annehmbare Salze oder ein Gemisch davon umfasst.

2. Kombination nach Anspruch 1, worin die Menge des mindestens einen aus Kartoffeln erhaltenen Protease-Inhibitors oder eines Gemisches davon zwischen 0,1% und 20% der Kombination, vorzugsweise zwischen 5 bis 15%, noch bevorzugter zwischen 4 bis 10% der Kombination beträgt.

3. Kombination nach Anspruch 1, worin die Menge von Dexpanthenol, dessen pharmazeutisch annehmbarer Salze oder eines Gemisch davon zwischen 0,01% und 40% der Kombination, vorzugsweise zwischen 2 bis 25%, noch bevorzugter zwischen 4 bis 25% der Kombination beträgt.

4. Kombination nach Anspruch 1 bis 3 zur Verwendung bei der Vorbeugung und/oder der Behandlung von durch Urin und/oder Fäzes verursachten Hautschäden und als Adjuvanz bei der Hautregeneration.

5. Kombination zur Verwendung nach Anspruch 4, worin die Hautschäden perineale, perianale Entzündungen oder Frühgeborenen-Dermatitis, Dermatitis bei Babys oder bei erwachsenen Patienten sind.

6. Kombination nach einem der Ansprüche 1 bis 3 oder Kombination zur Verwendung nach einem der Ansprüche 4 bis 5,
**dadurch gekennzeichnet, dass** diese einen weiteren zusätzlichen aktiven Wirkstoff umfasst, ausgewählt unter Antimykotika, Antiseptika und Desinfektionsmitteln, oberflächenaktiven Stoffen und Entschäumern, hautberuhigenden Substanzen, Entzündungshemmern, antibakteriellen Substanzen, Antihistaminika und Antioxidantien und/oder Vitaminen.

7. Pharmazeutische Zusammensetzung, welche die Kombinationen nach Ansprüchen 1 bis 3 zusammen mit pharmazeutisch annehmbaren Exzipienten und/oder Adjuvanzien umfasst.

8. Pharmazeutische Zusammensetzung nach Anspruch 7, **dadurch gekennzeichnet, dass** diese von 0,1 % bis 50%, vorzugsweise von 0,01 % bis 20% der Kombination umfasst.

9. Pharmazeutische Zusammensetzung nach Ansprüchen 7 oder 8 zur Verwendung bei der Vorbeugung und/oder der Behandlung von durch Urin und/oder Fäzes verursachten Hautschäden und als Adjuvanz bei der Hautregeneration.

10. Pharmazeutische Zusammensetzung nach Ansprüchen 7 oder 8, **dadurch gekennzeichnet, dass** diese zur gleichzeitigen, getrennten oder zur aufeinander folgenden Verwendung bei der Vorbeugung und/oder der Behandlung von durch Urin und/oder Fäzes verursachten Hautschäden und als Adjuvanz bei der Hautregeneration ist.

11. Pharmazeutische Zusammensetzung zur Verwendung nach Anspruch 9 **dadurch gekennzeichnet, dass** diese topisch verabreicht wird.

12. Pharmazeutische Zusammensetzung zur Verwendung nach Anspruch 11, worin die pharmazeutische Form ausgewählt ist unter einer Creme, einer Salbe, einem Gel, einem Schaum, einem Pulver, einem Spray und einem Verband, vorzugsweise einer Creme oder eine Salbe.

## Revendications

1. Combinaison comprenant au moins un inhibiteur de protéase dérivé de pomme de terre et du dexpanthénol, ses sels pharmaceutiquement acceptables, ou un mélange de ceux-ci.

2. Combinaison selon la revendication 1, dans laquelle la quantité dudit au moins un inhibiteur de protéase dérivé de pomme de terre ou d'un mélange de tels inhibiteurs est comprise entre 0,1 % et 20 % de la combinaison, de préférence entre 5 et 15 %, mieux encore entre 4 et 10 % de la combinaison.

3. Combinaison selon la revendication 1, dans laquelle la quantité de dexapanthénol, de ses sels pharmaceutiquement acceptables ou d'un de leurs mélanges est comprise entre 0,01 % et 40 % de la combinaison, de préférence entre 2 et 25 %, mieux encore entre 4 et 25 % de la combinaison.

4. Combinaison selon les revendication 1 à 3, pour une utilisation dans la prévention et/ou le traitement de dommages cutanés dus à l'urine et/ou aux fèces et en tant qu'adjuvant de réparation cutanée.

5. Combinaison pour une utilisation selon la revendication 4, dans laquelle lesdits dommages cutanés sont une inflammation ou une dermatite périnéale ou périanale présente chez les bébés prématurés, les bébés ou d'autres patients adultes.

6. Combinaison selon les revendications 1 à 3 ou combinaison pour une utilisation selon la revendication 4 ou 5, **caractérisée en ce qu'**elle comprend un autre principe actif additionnel, choisi parmi les antimycotiques, les antiseptiques et les désinfectants, les tensioactifs et les anti-moussants, les agents cutanés calmants, les anti-inflammatoires, les antibactériens, les antihistaminiques et les antioxydants et/ou les vitamines.

7. Composition pharmaceutique comprenant les combinaisons des revendications 1 à 3 conjointement avec des excipients et/ou adjuvants pharmaceutiquement acceptables.

8. Composition pharmaceutique selon la revendication 7, **caractérisée en ce qu'**elle contient de 0,1 % à 50 %, de préférence de 0,01 % à 20 %, de ladite combinaison.

9. Composition pharmaceutique selon la revendication 7 ou 8, pour une utilisation dans la prévention et/ou le traitement de dommages cutanés dus à l'urine et/ou aux fèces et en tant qu'adjuvant de réparation cutanée.

10. Composition pharmaceutique selon la revendication 7 ou 8, **caractérisée en ce qu'**elle est destinée à une utilisation simultanée, séparée ou séquentielle dans la prévention et/ou le traitement de dommages cutanés dus à l'urine et/ou aux fèces et en tant qu'adjuvant de réparation cutanée.

11. Composition pharmaceutique pour une utilisation selon la revendication 9, **caractérisée en ce qu'**elle est administrée par voie topique.

12. Composition pharmaceutique pour une utilisation selon la revendication 11, dans laquelle la forme pharmaceutique est choisie parmi une crème, une pommade, un gel, une mousse, une poudre, un spray et une gaze, de préférence une crème ou une pommade.
